(19) Europäisches Patentamt
European Patent Office
Office européen des brevets

(11) Publication number: **0 377 967 A2**

# (12) EUROPEAN PATENT APPLICATION

(21) Application number: **89312851.2**

(22) Date of filing: **08.12.89**

(51) Int. Cl.[5]: **C07D 498/08, C07D 513/08, A61K 31/535, A61K 31/54, C07D 519/00, //(C07D498/08, 265:00,221:00),(C07D513/08, 279:00,221:00),(C07D519/00, 498:00,471:00),(C07D519/00, 513:00,471:00)**

Claims for the following Contracting State: ES.

The title of the invention has been amended (Guidelines for Examination in the EPO, A-III, 7.3).

(30) Priority: **13.12.88 GB 8829079**

(43) Date of publication of application: **18.07.90 Bulletin 90/29**

(84) Designated Contracting States: **AT BE CH DE ES FR GB GR IT LI LU NL SE**

(71) Applicant: **BEECHAM GROUP PLC**
**Beecham House Great West Road**
**Brentford Middlesex TW8 9BD(GB)**

(72) Inventor: **King, Francis David Beecham**
**Phar.Med.Res.**
**Coldharbour Road The Pinnacles**
**Fourth Avenue Harlow Essex CM19 5AD(GB)**
Inventor: **Gregory, Julian Anthony**
**18 Osbourne Street**
**Mouldgreen Huddersfield(GB)**

(74) Representative: **Jones, Pauline et al**
**Beecham Pharmaceuticals Patents & Trade Marks Dept. Great Burgh Yew Tree Bottom Road**
**Epsom Surrey KT18 5XQ(GB)**

(54) **9-Aza-3-(oxa/thia)-bicyclo[3,2,1]nonane derivatives, process and intermediates for their preparation and pharmaceutical compositions containing them.**

(57) Compounds of formula (I) and pharmaceutically acceptable salts thereof:

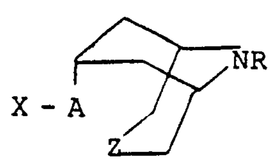

(I)

wherein
X is a phenyl group or a monocyclic 5 or 6 membered heteroaryl group, either of which group is optionally fused to a saturated or unsaturated 5-7 membered carbocyclic or heterocyclic ring;
A is a linking moiety;
Z is oxygen or sulphur; and
R is methyl or ethyl;
having 5-$HT_3$ receptor antagonist activity.

EP 0 377 967 A2

## NOVEL COMPOUNDS

This invention relates to novel compounds having pharmacological activity, to a process and intermediates for their preparation, and to their use as pharmaceuticals.

EP-A-41817 (Beecham Group p.l.c.) describes a group of exo-9-aza-3-oxabicyclo[3.2.1]non-7-yl amide derivatives.

EP-A-158265, EP-A-200444, EP-A-247266, EP-A-235878, EP-A-254584, EP-A-255297, EP-A-289170 and EP-A-315390 (Beecham Group p.l.c.), EP-A-158532 (A.H. Robins Company, Inc.), EP-A-67770 (Merrell Toraude et Compagnie), GB 2125398A and GB 2145416A (Sandoz Limited), EP-A-322016 (Duphar international Research B.V.), EP-A-307172 (Eli Lilly and Company), EP-A-323077, EP-A-306148 and GB 2208385A (John Wyeth and Brother Limited), EP-A-234872 (Adria Laboratories Inc.), EP-A-294292 (Adir et Compagnie), EP-A-339950 (Rorer International (overseas), Inc.), EP-A-309423 (Instituto de Angeli S.p.A.), EP-A-313393 (Yoshitomi Pharmaceutical industries Limited) and EP-A-328200 (Merck Sharp and Dohme Limited) disclose classes of compounds which have a saturated azabicyclic moiety, such as tropanyl, granatyl or quinuclidinyl, and are 5-$HT_3$ receptor antagonists.

A class of novel compounds has now been discovered in which the saturated azabicyclic moiety is endo-9-aza-3-(oxa/thia)-bicyclo[3.2.1]non-7-yl. These compounds have 5-$HT_3$ receptor antagonist activity.

Accordingly, the present invention provides a compound of formula (I), or a pharmaceutically acceptable salt thereof:

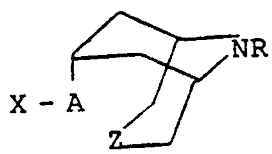

(I)

wherein

X is a phenyl group or a monocyclic 5 or 6 membered heteroaryl group, either of which group is optionally fused to a saturated or unsaturated 5-7 membered carbocyclic or heterocyclic ring;

A is a linking moiety;

Z is oxygen or sulphur; and

R is methyl or ethyl;

having 5-$HT_3$ receptor antagonist activity.

X may be unsubstituted or substituted, usually by one or more substituents selected from halogen, $C_{1-6}$ alkoxy, $C_{1-6}$ alkylthio, $C_{1-6}$ alkyl, hydroxy, amino, $C_{1-6}$ alkylamino, $C_{1-7}$ alkanoylamino, or two substituents on X (when fused), may be linked to form a saturated or unsaturated optionally substituted carbocyclic ring.

Heteroatoms for heteroaryl and heterocyclic groups are selected from oxygen, nitrogen and sulphur.

X may be joined to A by an aromatic carbon atom, or (when X is fused), by a carbocyclic ring carbon atom, or by a heterocyclic ring carbon or nitrogen atom.

When X is fused, and A is attached at an aromatic carbon atom, it is preferably attached at the aromatic carbon adjacent a 'fused' carbon atom, which is attached to the heteroatom of a heterocyclic ring in formula (I).

X may also be further joined to A as defined in formula (IA) hereinafter, when Y-$R_{10}$ is N-B=N.

Suitable examples of X are as described in the aforementioned patent publications relating to 5-$HT_3$ receptor antagonists, the subject matter of which is incorporated herein by reference.

Suitable examples of A include CONH (amide), COO (ester), NHCONH (ureide), CONHCONH (extended ureide), or a group of structure (h):

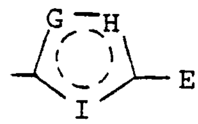

(h)

wherein the dotted circle represents two double bonds in any position in the 5 membered ring; two of G, H and I are selected from oxygen, sulphur, nitrogen and carbon and the other is oxygen, sulphur or nitrogen; and E is a bond or $C_{1-5}$ alkylene optionally substituted by phenyl or hydroxy.

For the avoidance of doubt, the suitable X values in formula (I) which are described in the referenced patent publications, are that part of the structure remaining when the saturated azabicyclic moiety and A (where A is one of the suitable examples listed above), are disregarded.

In a particular aspect, the present invention provides a compound of formula (IA), or a pharmaceutically acceptable salt thereof:

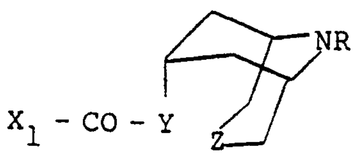

(IA)

wherein

Y is NH or O (or is joined to $R_{10}$ as defined below);

X is a group of formula (a), (b), (c), (d), (e), (f) or (g):

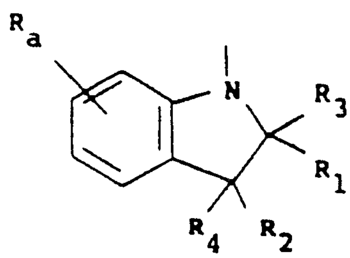

(a)

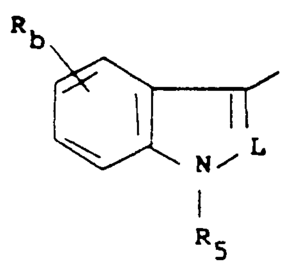

(b)

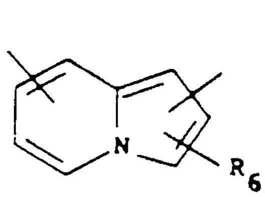

(c)

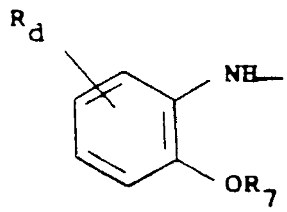

(d)

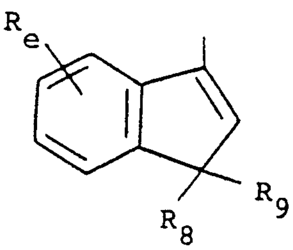

(e)

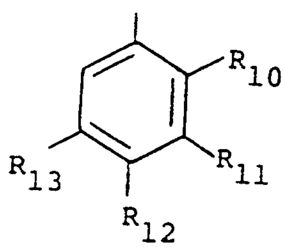

(f)

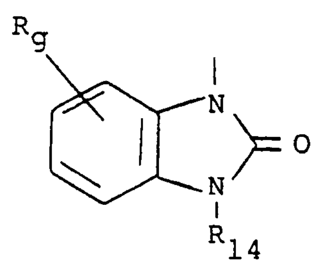

(g)

wherein

$R_a$ to $R_e$ and $R_g$ are selected from hydrogen, halogen or hydroxy;

$R_1$ is hydrogen and $R_2$ is hydrogen or $C_{1-4}$ alkyl; or

$R_1$ and $R_2$ together are a bond;

$R_3$ to $R_7$ are independently hydrogen or $C_{1-6}$ alkyl; and

$R_4$ together with $R_2$ may be $C_{2-7}$ polymethylene when $R_1$ is hydrogen;

$R_8$ and $R_9$ are independently selected from hydrogen or $C_{1-6}$ alkyl or $R_8$ and $R_9$ together are $C_{2-6}$

polymethylene or $C_{2-5}$ polymethylene interrupted by an -O- linkage;
either $R_{10}$ is $C_{1-6}$ alkoxy or $R_{10}$ is joined to Y so that Y-$R_{10}$ is N-B=N where B is N or CH;
$R_{11}$ is hydrogen;
$R_{12}$ is amino optionally substituted by a $C_{1-6}$ alkyl group, or $C_{1-7}$ alkanoylamino; and
$R_{13}$ is halo or $C_{1-6}$ alkylthio; or
$R_{10}$ is hydrogen;
$R_{11}$ is halo, $C_{1-6}$ alkoxy or $C_{1-6}$ alkyl;
$R_{12}$ is hydrogen or $C_{1-6}$ alkoxy; and
$R_{13}$ is halo, $C_{1-6}$ alkoxy or $C_{1-6}$ alkyl;
$R_{14}$ is hydrogen or $C_{1-6}$ alkyl;
L is CH or N;
Z is O or S; and
R is methyl or ethyl.

Examples of moieties in alkyl or alkyl containing groups in $R_1$ to $R_{14}$ include methyl, ethyl, n- and iso-propyl, n-, iso-, sec- and tert-butyl, preferably methyl.

Suitable examples of $R_2$ and $R_4$ or $R_8$ and $R_9$ when joined include $C_2$, $C_3$, $C_4$, $C_5$ or $C_6$ polymethylene, preferably $C_2$, $C_3$, $C_4$ or $C_5$ polymethylene.

$R_a$ to $R_e$ and $R_g$ are preferably selected from hydrogen, fluoro, chloro and hydroxy, most preferably hydrogen. $R_b$ may be 5-, 6- or 7-chloro or fluoro.

When X is of sub-formula (a), one of $R_1$ and $R_3$ is preferably hydrogen and one or both of $R_2$ and $R_4$ - (most preferably both) are alkyl groups, such as methyl, or are joined to form $C_{2-7}$ polymethylene; or when one of $R_2$ and $R_4$ is hydrogen, the other is preferably ethyl or n- or iso- propyl.

When X is of sub-formula (b), $R_5$ is preferably hydrogen or a methyl or ethyl group.

When X is of sub-formula (c), one of CO-Y and $R_6$ is attached at the 1-position and the other is attached at the 3-position as depicted in sub-formula (c), and $R_6$ is preferably methyl or ethyl.

When X is of sub-formula (d), $R_7$ is preferably methyl.

When X is of sub-formula (e), $R_8$ and $R_9$ are preferably both methyl groups.

When X is of sub-formula (f), and $R_{10}$ is $C_{1-6}$ alkoxy or is joined to Y, $R_{12}$ is preferably amino and $R_{13}$ is preferably chloro or bromo, most preferably chloro. $R_{10}$ is preferably methoxy when $C_{1-6}$ alkoxy.

When X is of sub-formula (f), and $R_{10}$ is hydrogen, $R_9$ and $R_{11}$ are preferably chloro or methyl and $R_{10}$ is preferably hydrogen.

When X is of sub-formula (g), $R_{14}$ is preferably hydrogen or methyl.

X is preferably a group of sub-formula (e).

Y is preferably NH.

The pharmaceutically acceptable salts of the compounds of the formula (I) include acid addition salts with conventional acids such as hydrochloric, hydrobromic, boric, phosphoric, sulphuric acids and pharmaceutically acceptable organic acids such as acetic, tartaric, maleic, citric, succinic, benzoic, ascorbic, methanesulphonic, α-keto glutaric, α-glycerophosphoric, and glucose-1-phosphoric acids.

The pharmaceutically acceptable salts of the compounds of the formula (I) are usually acid addition salts with acids such as hydrochloric, hydrobromic, phosphoric, sulphuric, citric, tartaric, lactic and acetic acid.

Preferably the acid addition salt is the hydrochloride salt.

Examples of pharmaceutically acceptable salts include quaternary derivatives of the compounds of formula (I) such as the compounds quaternised by compounds $R_x$-T wherein $R_x$ is $C_{1-6}$ alkyl, phenyl-$C_{1-6}$ alkyl or $C_{5-7}$ cycloalkyl, and T is a radical corresponding to an anion of an acid. Suitable examples of $R_x$ include methyl, ethyl and n- and iso-propyl; and benzyl and phenethyl. Suitable examples of T include halide such as chloride, bromide and iodide.

Examples of pharmaceutically acceptable salts also include internal salts such as N-oxides.

The compounds of the formula (I), their pharmaceutically acceptable salts, (including quaternary derivatives and N-oxides) may also form pharmaceutically acceptable solvates, such as hydrates, which are included wherever a compound of formula (I) or a salt thereof is herein referred to.

It will of course be realised that some of the compounds of the formula (I) have chiral or prochiral centres and thus are capable of existing in a number of stereoisomeric forms including enantiomers. The invention extends to each of these stereoisomeric forms (including enantiomers), and to mixtures thereof (including racemates). The different stereoisomeric forms may be separated one from the other by the usual methods.

The invention also provides a process for the preparation of a compound of formula (I) which process comprises reacting a compound $X'$-$A_1$ with a compound of formula (II):

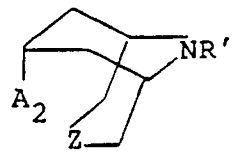

(II)

wherein $A_1$ and $A_2$ are moieties which react together, usually by an amide or ester coupling, or by condensation to form a heterocycle (h) as hereinbefore defined, to form A as defined; X′ is X or a group convertible thereto and R′ is R as defined or a hydrogenolysable protecting group; and thereafter as desired or necessary, converting X′ to X, converting R′, when other than R, to R, and optionally forming a pharmaceutically acceptable salt of the compound of formula (I).

Suitable values of $A_1$ and $A_2$ are as described in the aforementioned patent publications.

Intermediates of the formula X′-$A_1$ are generally known from the aforementioned patent publications, or are prepared by analogous methods to those used for structurally related known compounds.

Intermediates of the formula (II) are generally prepared from the compound of formula (III):

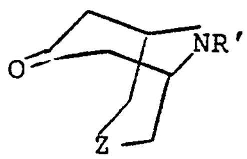

(III)

The intermediate of formula (III) wherein Z is oxygen and R′ is methyl is prepared by C.L. Zirkle et. al., J. Org. Chem. 26, 395, 1961.

In a particular aspect, the invention also provides a process for the preparation of a compound of formula (IA), or a pharmaceutically acceptable salt thereof, which process comprises reacting a compound of formula (IV):

$X_1'$-$COQ_1$ (IV)

with a compound of formula (V):

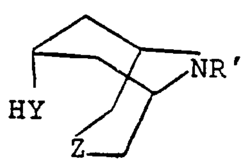

(V)

or a reactive derivative thereof, when Y is O;

wherein $X_1'$ is $X_1$ or a group convertible thereto; $Q_1$ is a leaving group; R′ is R as defined, or a hydrogenolysable protecting group; and the remaining variables are as hereinbefore defined; and thereafter optionally converting $X_1'$ to $X_1$, including any $R_a$, $R_b$, $R_c$, $R_d$, $R_e$, $R_g$ or $R_{10}$, $R_{11}$, $R_{12}$, $R_{13}$, $R_{14}$ group to another such group, converting R′, when other than R, to R; and optionally forming a pharmaceutically acceptable salt of the resultant compound of formula (IA).

Examples of leaving groups $Q_1$, displaceable by a nucleophile, include halogen such as chloro and bromo, $C_{1-4}$ alkoxy, such as $CH_3O$ and $C_2H_5O$-, PhO-, or activated hydrocarbyloxy, such as $Cl_5C_6O$- or $Cl_3CO$-

If a group $Q_1$ is a halide, then the reaction is preferably carried out at non-extreme temperatures in an inert non-hydroxylic solvent, such as benzene, dichloromethane, toluene, diethyl ether, tetrahydrofuran (THF) or dimethylformamide (DMF). It is also preferably carried out in the presence of an acid acceptor, such as an organic base, in particular a tertiary amine, such as triethylamine, trimethylamine, pyridine or picoline, some of which can also function as the solvent. Alternatively, the acid acceptor can be inorganic, such as calcium carbonate, sodium carbonate or potassium carbonate. Temperatures of 0°-100°C, in particular 10-80°C are suitable.

If a group $Q_1$ is $C_{1-4}$ alkoxy, phenoxy or activated hydrocarbyloxy then the reaction is preferably carried out in an inert polar solvent, such as toluene or dimethylformamide. It is also preferred that the group $Q_1$ is $Cl_3CO$- and that the reaction is carried out in toluene at reflux temperature.

When Y is O the compound of formula (V) may be in the form of a reactive derivative thereof, which is often a salt, such as the lithium, sodium or potassium salt.

Usually, $X_1'$ will be $X_1$, but when $R_{10}$ is joined to Y, in formula (IA), $X_1'$ is of sub-formula (f) wherein $R_{10}$ is nitro or amino, which may be subsequently be linked to Y as described in EP-A-315390.

It will be apparent that compounds of the formula (IA) containing an $R_a$ to $R_e$, $R_g$ or $R_{10}$ to $R_{14}$ group which is convertible to another such group are useful novel intermediates. i.e. a hydrogen substituent is convertible to a halogen substituent by halogenation using conventional halogenating agents; or a $C_{1-7}$ alkanoylamino substituent is convertible to amino by conventional hydrolysis.

$R'$ when other than R may be a hydrogenolysable protecting group which is benzyl optionally substituted by one or two groups selected from halo, $C_{1-4}$ alkoxy and $C_{1-4}$ alkyl. Such benzyl groups may, for example, be removed, when $R_a$ to $R_e$, $R_g$, $R_{11}$ to $R_{14}$ is not halogen, by conventional transition metal catalysed hydrogenolysis to give compounds of the formula (VI):

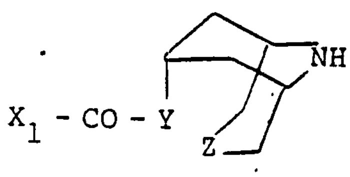

(VI)

wherein the variables are as defined in formula (IA).

This invention also provides a further process for the preparation of a compound of the formula (IA) or a pharmaceutically acceptable salt thereof, which comprises N-alkylating a compound of formula (VI), and optionally forming a pharmaceutically acceptable salt of the resulting compound of the formula (IA).

In this further process of the invention 'N-alkylation' comprises the substitution of the N-atom depicted in formula (VI) by a group R as hereinbefore defined. This may be achieved by reaction with a compound $RQ_3$ wherein R is as hereinbefore defined and $Q_3$ is a leaving group.

Suitable values for $Q_3$ include groups displaced by nucleophiles such as Cl, Br, I, $OSO_2CH_3$ or $OSO_2C_6H_4pCH_3$.

Favoured values for $Q_3$ include Cl, Br and I.

The reaction may be carried out under conventional alkylation conditions for example in an inert solvent such as dimethylformamide in the presence of an acid acceptor such as potassium carbonate. Generally the reaction is carried out at non-extreme temperature such as at ambient or slightly above.

Alternatively, 'N-alkylation' may be effected under conventional reductive alkylation conditions.

Interconverting R in the compound of the formula (V) before coupling with the compound of the formula (IV) is also possible. Such interconversions are effected conveniently under the above conditions. It is desirable to protect any amine function with a group readily removable by acidolysis such as a $C_{2-7}$ alkanoyl group, before R interconversion.

It is often convenient in the preparation of such a compound of formula (V) to prepare the corresponding compound wherein the methylene group is replaced by -CO-, or for R is methyl, where the methyl group is replaced by alkoxycarbonyl. Such compounds may then be reduced using a strong reductant such as lithium aluminium hydride to the corresponding compound of formula (IV).

The compounds of formula (IV) are known or are preparable analogously to, or routinely from, known compounds.

Compounds of the formula (V) wherein $R'$ is R as defined, other than the compound wherein Y is O, Z is O and $R'$ is methyl, are novel and form an aspect of the invention.

Compounds of the formula (I) may also be prepared by the processes described in the aforementioned European Patent Publications.

It will be realised that in the compound of the formula (I) the -A- linkage has an endo orientation with respect to the ring of the bicyclic moiety to which it is attached. A mixture of endo and exo isomers of the compound of the formula (I) may be synthesised non-stereospecifically and the desired isomer separated conventionally therefrom e.g. by chromatography; or alternatively the endo isomer may if desired by synthesised from the corresponding endo form of the compound of the formula (II).

Pharmaceutically acceptable salts of the compounds of this invention may be formed conventionally.

The salts may be formed for example by reaction of the base compound of formula (I) with a pharmaceutically acceptable organic or inorganic acid.

The compounds of the present invention are 5-$HT_3$ receptor antagonists and it is thus believed may generally be used in the treatment or prophylaxis of migraine, cluster headaches and trigeminal neuralgia; visceral pain; and also as anti-emetics, in particular that of preventing vomiting and nausea associated with cancer therapy, and motion sickness. Examples of such cancer therapy include that using cytotoxic agents, such as cisplatin, doxorubicin and cyclophosphamide, particularly cisplatin; and also radiation treatment. Compounds which are 5-$HT_3$ receptor antagonists may also be of potential use in the treatment of CNS disorders such as anxiety, psychosis, senile dementia and drug dependence; arrhythmia, obesity and gastrointestinal disorders, such as irritable bowel syndrome.

The invention also provides a pharmaceutical composition comprising a compound of formula (I), or a pharmaceutically acceptable salt thereof, and a pharmaceutically acceptable carrier.

Such compositions are prepared by admixture and are usually adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art, for example with an enteric coating.

Suitable fillers for use include cellulose, mannitol, lactose and other similar agents. Suitable disintegrants include starch, polyvinylpolypyrrolidone and starch derivatives such as sodium starch glycollate. Suitable lubricants include, for example, magnesium stearate.

Suitable pharmaceutically acceptable wetting agents include sodium lauryl sulphate. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral liquid preparations are usually in the form of aqueous or oily suspensions, solutions, emulsions, syrups, or elixirs or are presented as a dry product for reconstitution with water or other suitable vehicle before use. Such liquid preparations may contain conventional additives such as suspending agents, emulsifying agents, non-aqueous vehicles (which may include edible oils), preservatives, and flavouring or colouring agents.

The oral compositions may be prepared by conventional methods of blending, filling or tabletting. Repeated blending operations may be used to distribute the active agent throughout those compositions employing large quantities of fillers. Such operations are, of course, conventional in the art.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure of ethylene oxide before suspending in the sterile vehicle. Advantageously, a surfactant or wetting agent is included in the composition to facilitate uniform distribution of the compound of the invention.

The invention further provides a method of treatment or prophylaxis of migraine, cluster headache, trigeminal neuralgia, visceral pain, CNS disorders and/or emesis in mammals, such as humans, which comprises the administration of an effective amount of a compound of the formula (I) or a pharmaceutically acceptable salt thereof.

An amount effective to treat the disorders hereinbefore described depends on the relative efficacies of the compounds of the invention, the nature and severity of the disorder being treated and the weight of the

mammal. However, a unit dose for a 70kg adult will normally contain 0.05 to 1000mg for example 0.5 to 500mg, of the compound of the invention. Unit doses may be administered once or more than once a day, for example, 2, 3 or 4 times a day, more usually 1 to 3 times a day, that is in the range of approximately 0.0001 to 50mg/kg/day, more usually 0.0002 to 25 mg/kg/day.

No adverse toxicological effects are indicated at any of the aforementioned dosage ranges.

The invention also provides a compound of formula (I) or a pharmaceutically acceptable salt thereof for use as an active therapeutic substance, in particular for use in the treatment of migraine, cluster headache, trigeminal neuralgia, visceral pain, CNS disorders and/or emesis.

The following Examples illustrate the preparation of compounds of formula (I); the following descriptions illustrate the preparation of intermediates.

Description 1

9-Aza-3-oxa-9-methylbicyclo[3.3.1]nonan-7-one

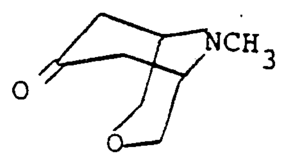

(D1)

Acetic acid (6ml) was added to a suspension of oxydiacetaldehyde bis(diethyl acetal) (23.2g) (J. Org. Chem, 1961, 26, 395) in water (22ml) and heated to reflux for 45 min. The cooled solution, acetone dicarboxylic acid (35.0g) and methylamine hydrochloride (14.5g) were added to a solution of disodium orthophosphate (94.5g) and citric acid (24.6g) in water (300ml). The pH was adjusted to 5-5.5 using 40% NaOH (aq), the total reaction volume made up to 600ml and the reaction stirred for 2 days.

The reaction was acidified to pH 1 and washed with ether. The aqueous layer was basified to pH 14 and continuously extracted with dichloromethane overnight. The organic layer was dried ($Na_2SO_4$), filtered and the solvent evaporated. The residue was filtered through basic alumina using a gradient elution of dichloromethane to chloroform to give the title ketone (5.9g).

Description 2

9-Aza-3-oxa-9-methylbicvclo[3.3.1]nonan-7-one oxime hydrochloride (D2)

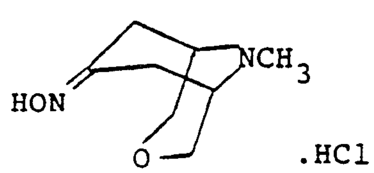

(D2)

Hydroxylamine hydrochloride (2.8g) was added to a stirred solution of D1 (5.9g) in ethanol (125ml) and heated to reflux for 2h. The reaction was allowed to cool, ether added and the precipitate (6.7g) collected.

NMR ($d_6$-DMSO, 270MHz) δ:

11.6 (s, 1H), 10.55 (s, 1H), 2.4-4.2 (m, 9H), 3.35 (s, 3H).

Description 3

endo-7-Amino-9-aza-3-oxa-9-methylbicyclo[3.3.1]nonane (D3)

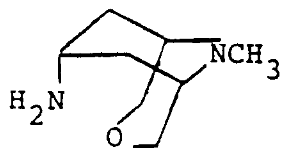

(D3)

Concentrated sulphuric acid (1.9ml) was added dropwise to a suspension of $LiAlH_4$ (1.9g) in THF (125ml), cooled to 0° C under $N_2$ and stirred for 1h. A solution of D2 (free base, 2.9g) in THF (75ml) was added dropwise and the reaction heated to reflux overnight. The reaction was quenched by sequential dropwise addition of water (1.9ml), 10% NaOH (aq) (2.9ml) and water (5.8ml), then stirred until a white precipitate formed. The reaction was filtered and the solvent evaporated. The residue was distilled under reduced pressure to yield the amine (0.56g) 160-170° C, 5mmHg.
MS 156 ($M^+$, 40%), 140 ($M-NH_2$, 15), 125 ($M-HO=CH_2$, 35).

Example 1

endo-4-Acetamido-5-chloro-2-methoxy-N-(9-methyl-9-aza-3-oxabicyclo[3.3.1]nonan-7-yl)benzamide (E1)

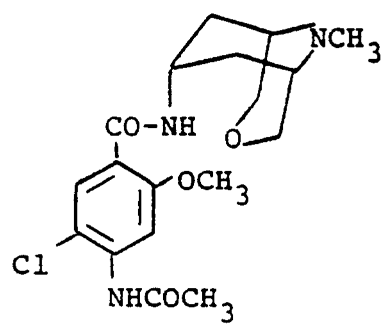

(E1)

To a stirred solution of 4-acetamido-5-chloro-2-methoxybenzoyl chloride (0.88g) in dichloromethane (25ml) at 0° C, was added dropwise a solution of D3 (0.56g) and $Et_2N$ (0.5ml) in dichloromethane (10ml) and the reaction stirred overnight under $N_2$. The reaction was washed with saturated $NaHCO_3$ (aq), dried ($Na_2SO_4$), filtered and the solvent evaporated. The residue was filtered through basic alumina using a gradient elution of dichloromethane through to 2% methanol/chloroform to yield the amide (0.74g).

Example 2

endo-4-Amino-5-chloro-2-methoxy-N-(9-methyl-9-aza-3-oxabicyclo[3.3.1]nonan-7-yl)benzamide (E2)

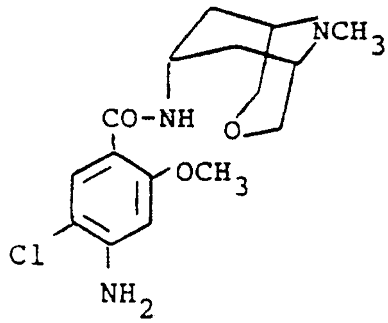

(E2)

To a solution of E1 (0.74g) in ethanol (25ml) was added 10% NaOH (aq) (1.9ml) and the reaction mixture heated under reflux for 2h. The solvent was evaporated and the residue taken up in water and extracted into dichloromethane. The organic layer was dried, filtered and the solvent evaporated. The residue was filtered through basic alumina using a gradient elution of dichloromethane through to chloroform. The crude product was crystallised as the hydrochloride salt (0.13g).

M.p. 190-198 °C (EtOH/$Et_2O$)

NMR ($CDCl_3$, 270MHz) δ:

8.1 (s, 1H), 6.28 (s, 1H), 4.8 (q, 1H), 4.32 (s, 2H), 3.99 (d, 2H), 3.83 (s, 3H), 3.7-3.9 (m, 2H), 2.4-2.75 (m, 4H), 2.54 (s, 3H), 1.45 (m, 2H)

MS: 339 ($M^+$, 42%), 307 (8), 268 (13), 184 ($ArCO^+$, 54), 94 (100)

Examples 3-5

The following compounds were prepared from D3 in an analogous manner to that described in EP-A-235878, EP-A-200444 and EP-A-247266, respectively.

endo-N-(9-Methyl-9-aza-3-oxabicyclo[3.3.1]nonan-7-yl)-N′-(2-methoxyphenyl)urea (E3)

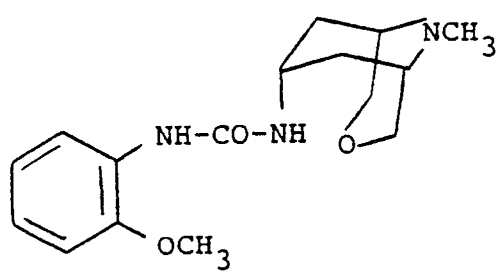

(E3)

m.p. 153-155 °C ($Et_2O$)

NMR ($CDCl_3$, 250MHz) δ:

8.02-8.10 (m, 1H), 7.20 (brd, 1H), 6.80-6.98 (m, 3H), 6.65 (brs, 1H), 4.40 (dt, 1H), 3.98 (brd, 2H), 3.88 (s, 3H), 3.75 (d, 2H), 2.53 (brs, 2H), 2.40-2.55 (m, 5H including 2.52, s, 3H), 1.46 (d, 2H)

endo-N-(9-Methyl-9-aza-3-oxabicyclo[3.3.1]nonan-7-yl)-1-methyl-1H-indazol-3-carboxamide (E4)

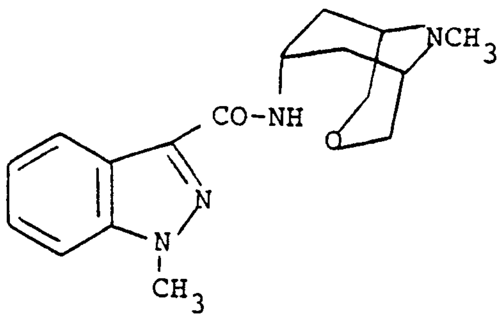

(E4)

m.p. 212-220° C
NMR ($CDCl_3$, 250MHz) δ:
9.32 (brd, 1H), 8.39 (d, 1H), 7.20-7.45 (m, 3H), 4.80 (dt, 1H), 4.05-4.17 (m, 5H including 4.10 s, 3H), 3.90 (d, 2H), 2.76 (brs, 2H), 2.50-2.65 (m, 5H including 2.60, s, 3H), 1.58 (d, 2H)

endo-N-(9-Methyl-9-aza-3-oxabicyclo[3.3.1]nonan-7-yl)-3,3-dimethyl-1-indoline carboxamide (E5)

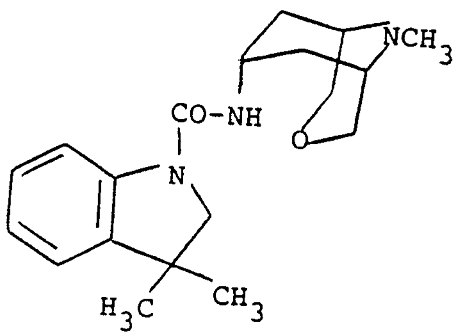

(E5)

m.p. 115-120° C
NMR ($CDCl_3$, 250MHz) δ:
7.94 (d, 1H), 7.49 (brd, 1H), 7.17 (t, 1H), 7.09 (d, 1H), 6.92 (t, 1H), 4.46 (dt, 1H), 4.05 (brd, 2H), 3.79 (brd, 2H), 3.58 (s, 1H), 2.72 (brs, 2H), 2.40-2.60 (m, 5H including 2.56, s, 3H), 1.50 (brd, 2H), 1.32 (s, 6H)

Pharmacology

Antagonism of the von Bezold-Jarisch reflex

The compounds were evaluated for antagonism of the von Bezold-Jarisch reflex evoked by 5-HT in the anaesthetised rat according to the following method:

Male rats 250-350g, were anaesthetised with urethane (1.25g/kg intraperitoneally) and blood pressure and heart rate recorded as described by Fozard J.R. et al., J. Cardiovasc. Pharmacol. 2, 229-245 (1980). A submaximal dose of 5-HT (usually 6µg/kg) was given repeatedly by the intravenous route and changes in heart rate quantified. Compounds were given intravenously and the concentration required to reduce the 5-HT-evoked response to 50% of the control response ($ED_{50}$) was then determined.

The compound of Example 2 gave an $ED_{50}$ value of 0.09 µg/kg i.v.

Compounds E3, E4 and E5 were active at a dose of 1µg/kg i.v.

**Claims**

1. A compound of formula (I) or a pharmaceutically acceptable salt thereof:

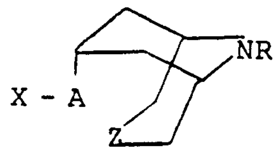

(I)

wherein
X is a phenyl group or a monocyclic 5 or 6 membered heteroaryl group, either of which group is optionally fused to a saturated or unsaturated 5-7 membered carbocyclic or heterocyclic ring;
A is a linking moiety;
Z is oxygen or sulphur; and
R is methyl or ethyl;
having $5\text{-}HT_3$ receptor antagonist activity.

2. A compound according to claim 1 wherein A is CONH, NHCONH, CONHCONH or a group of structure (h):

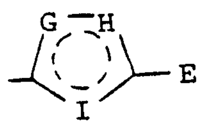

(h)

wherein the dotted circle represents two double bonds in any position in the 5 membered ring; two of G, H and I are selected from oxygen, sulphur, nitrogen and carbon and the other is oxygen, sulphur or nitrogen; and E is a bond or $C_{1-5}$ alkylene optionally substituted by phenyl or hydroxy.

3. A compound according to claim 1, of formula (IA), or a pharmaceutically acceptable salt thereof:

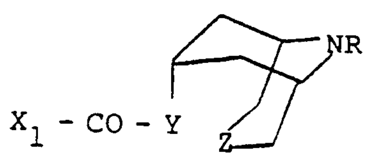

(IA)

wherein
Y is NH or O (or is joined to $R_{10}$ as defined below);
X is a group of formula (a), (b), (c), (d), (e), (f) or (g):

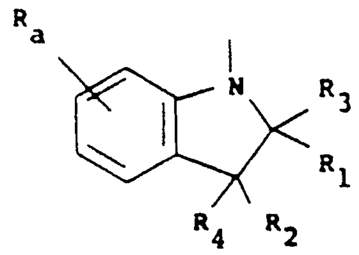

(a)

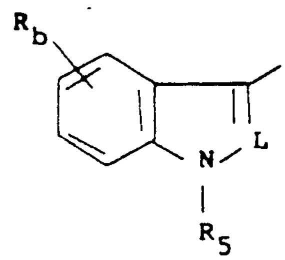

(b)

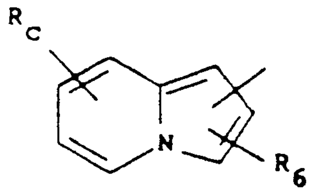

(c)

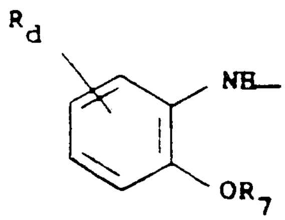

(d)

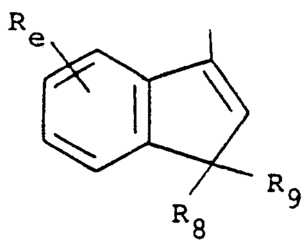

(e)

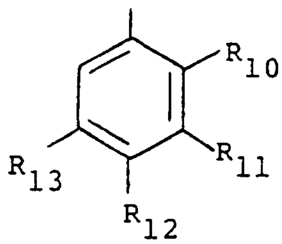

(f)

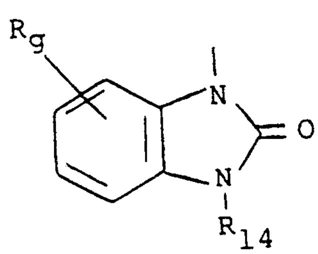

(g)

wherein
$R_a$ to $R_e$ and $R_g$ are selected from hydrogen, halogen or hydroxy;
$R_1$ is hydrogen and $R_2$ is hydrogen or $C_{1-4}$ alkyl; or
$R_1$ and $R_2$ together are a bond;
$R_3$ to $R_7$ are independently hydrogen or $C_{1-6}$ alkyl; and
$R_4$ together with $R_2$ may be $C_{2-7}$ polymethylene when $R_1$ is hydrogen;
$R_8$ and $R_9$ are independently selected from hydrogen or $C_{1-6}$ alkyl or $R_8$ and $R_9$ together are $C_{2-6}$

polymethylene or $C_{2-5}$ polymethylene interrupted by an -O- linkage;
either $R_{10}$ is $C_{1-6}$ alkoxy or $R_{10}$ is joined to Y so that Y-$R_{10}$ is N-B=N where B is N or CH;
$R_{11}$ is hydrogen;
$R_{12}$ is amino optionally substituted by a $C_{1-6}$ alkyl group, or $C_{1-7}$ alkanoylamino; and
$R_{13}$ is halo or $C_{1-6}$ alkylthio; or
$R_{10}$ is hydrogen;
$R_{11}$ is halo, $C_{1-6}$ alkoxy or $C_{1-6}$ alkyl;
$R_{12}$ is hydrogen or $C_{1-6}$ alkoxy; and
$R_{13}$ is halo, $C_{1-6}$ alkoxy or $C_{1-6}$ alkyl;
$R_{14}$ is hydrogen or $C_{1-6}$ alkyl;
L is CH or N;
Z is O or S; and
R is methyl or ethyl.

4. A compound according to claim 3 wherein X is of sub-formula (a), one of $R_1$ and $R_3$ is hydrogen and $R_2$ and $R_4$ are both $C_{1-6}$ alkyl groups or are joined to form $C_{2-7}$ polymethylene.

5. A compound according to claim 3 wherein X is of sub-formula (b), and $R_5$ is hydrogen or a methyl or ethyl group.

6. A compound according to claim 3 wherein X is of sub-formula (d) and $R_7$ is methyl.

7. A compound according to claim 3 wherein X is of sub-formula (f) wherein $R_{10}$ is methoxy, $R_{12}$ is amino and $R_{13}$ is chloro or bromo.

8. A compound according to any one of claims 1 to 7, wherein Z is O.

9. A compound selected from the group consisting of:
endo-4-amino-5-chloro-2-methoxy-N-(9-methyl-9-aza-3-oxabicyclo[3.3.1]nonan-7-yl)benzamide,
endo-N-(9-methyl-9-aza-3-oxabicyclo[3.3.1]nonan-7-yl)-N′-(2-methoxyphenyl)urea,
endo-N-(9-methyl-9-aza-3-oxabicyclo[3.3.1]nonan-7-yl)-1-methyl-1H-indazol-3-carboxamide,
endo-N-(9-methyl-9-aza-3-oxabicyclo[3.3.1]nonan-7-yl)-3,3-dimethyl-1-indoline carboxamide,
and pharmaceutically acceptable salts of any of the foregoing compounds.

10. A process for the preparation of a compound according to claim 1 which process comprises reacting a compound X′-$A_1$ with a compound of formula (II):

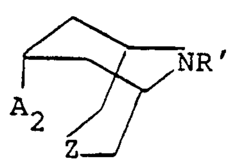

(II)

wherein $A_1$ and $A_2$ are moieties which react together, usually by an amide or ester coupling, or by condensation to form a heterocycle (h) as defined in claim 2, to form A as defined in claim 1; X′ is X or a group convertible thereto and R′ is R as defined in claim 1, or a hydrogenolysable protecting group; and thereafter as desired or necessary, converting X′ to X, converting R′, when other than R, to R, and optionally forming a pharmaceutically acceptable salt of the compound of formula (I).

11. A process for the preparation of a compound according to claim 3, which process comprises reacting a compound of formula (IV):
$X_1'$-$COQ_1$ (IV)
with a compound of formula (V):

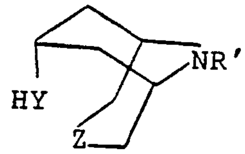

(V)

or a reactive derivative thereof, when Y is O;
wherein $X_1'$ is $X_1$ or a group convertible thereto; $Q_1$ is a leaving group; R′ is R as defined in claim 1, or a hydrogenolysable protecting group; and the remaining variables are as defined in claim 3; and thereafter

optionally converting $X_1'$ to $X_1$, including any $R_a$, $R_b$, $R_c$, $R_d$, $R_e$, $R_g$ or $R_{10}$, $R_{11}$, $R_{12}$, $R_{13}$, $R_{14}$ group to another such group, converting $R'$, when other than R, to R; and optionally forming a pharmaceutically acceptable salt of the resultant compound of formula (IA).

12. An intermediate of formula (V) wherein $R'$ is R, as defined in claim 11, other than the compound wherein Y is O, Z is O and $R'$ is methyl.

13. endo-7-Amino-9-aza-3-oxa-9-methylbicyclo[3.3.1]nonane.

14. A pharmaceutical composition comprising a compound according to any one of claims 1 to 9, and a pharmaceutically acceptable carrier.

15. A compound according to any one of claims 1 to 9, for use as an active therapeutic substance.

16. A compound according to any one of claims 1 to 9, for use in the treatment of migraine, cluster headache, trigeminal neuralgia, visceral pain, CNS disorders and/or emesis.

17. Use of a compound according to any one of claims 1 to 9, in the manufacture of a medicament for the treatment of migraine, cluster headache, trigeminal neuralgia, visceral pain, CNS disorders and/or emesis.

Claims for the following Contracting State : ES

1. A process for the preparation of a compound of formula (I) or a pharmaceutically acceptable salt thereof:

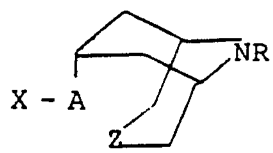

(I)

wherein
X is a phenyl group or a monocyclic 5 or 6 membered heteroaryl group, either of which group is optionally fused to a saturated or unsaturated 5-7 membered carbocyclic or heterocyclic ring;
A is a linking moiety;
Z is oxygen or sulphur; and
R is methyl or ethyl;
having 5-$HT_3$ receptor antagonist activity; which process comprises reacting a compound $X'$-$A_1$ with a compound of formula (II):

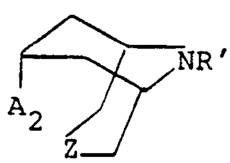

(II)

wherein $A_1$ and $A_2$ are moieties which react together, usually by an amide or ester coupling, or by condensation to form a heterocycle (h) as defined, to form A; $X'$ is X or a group convertible thereto and $R'$ is R as defined, or a hydrogenolysable protecting group; and thereafter as desired or necessary, converting $X'$ to X, converting $R'$, when other than R, to R, and optionally forming a pharmaceutically acceptable salt of the compound of formula (I).

2. A process according to claim 1 wherein A is CONH, NHCONH, CONHCONH or a group of structure (h):

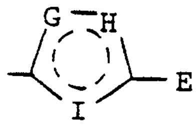

(h)

wherein the dotted circle represents two double bonds in any position in the 5 membered ring; two of G, H and I are selected from oxygen, sulphur, nitrogen and carbon and the other is oxygen, sulphur or nitrogen; and E is a bond or $C_{1-5}$ alkylene optionally substituted by phenyl or hydroxy.

3. A process for the preparation of a compound within formula (I) in claim 1, of formula (IA), or a pharmaceutically acceptable salt thereof:

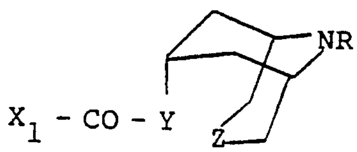

(IA)

wherein
Y is NH or O (or is joined to $R_{10}$ as defined below);
X is a group of formula (a), (b), (c), (d), (e), (f) or (g):

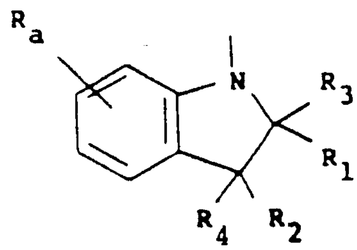

(a)

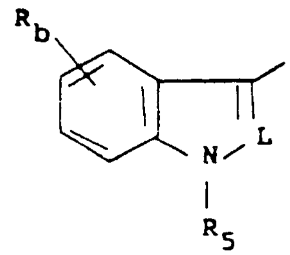

(b)

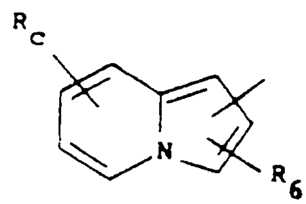

(c)

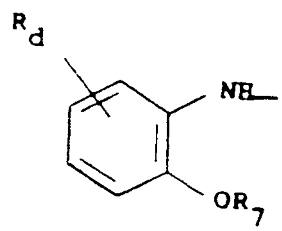

(d)

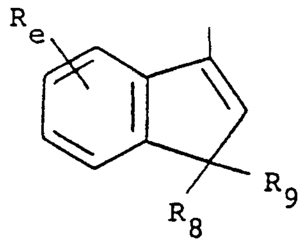

(e)

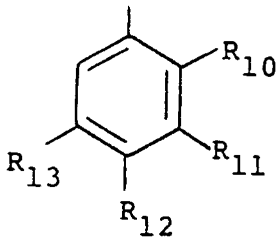

(f)

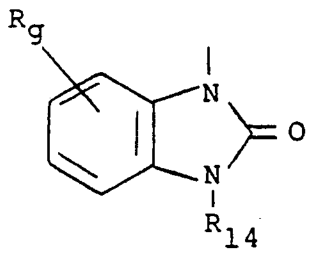

(g)

wherein

$R_a$ to $R_e$ and $R_g$ are selected from hydrogen, halogen or hydroxy;

$R_1$ is hydrogen and $R_2$ is hydrogen or $C_{1-4}$ alkyl; or

$R_1$ and $R_2$ together are a bond;

$R_3$ to $R_7$ are independently hydrogen or $C_{1-6}$ alkyl; and

$R_4$ together with $R_2$ may be $C_{2-7}$ polymethylene when $R_1$ is hydrogen;

$R_8$ and $R_9$ are independently selected from hydrogen or $C_{1-6}$ alkyl or $R_8$ and $R_9$ together are $C_{2-6}$ polymethylene or $C_{2-5}$ polymethylene interrupted by an -O- linkage;

either $R_{10}$ is $C_{1-6}$ alkoxy or $R_{10}$ is joined to Y so that Y-$R_{10}$ is N-B=N where B is N or CH;

$R_{11}$ is hydrogen;

$R_{12}$ is amino optionally substituted by a $C_{1-6}$ alkyl group, or $C_{1-7}$ alkanoylamino; and

$R_{13}$ is halo or $C_{1-6}$ alkylthio; or

$R_{10}$ is hydrogen;

$R_{11}$ is halo, $C_{1-6}$ alkoxy or $C_{1-6}$ alkyl;

$R_{12}$ is hydrogen or $C_{1-6}$ alkoxy; and

$R_{13}$ is halo, $C_{1-6}$ alkoxy or $C_{1-6}$ alkyl;

$R_{14}$ is hydrogen or $C_{1-6}$ alkyl;

L is CH or N;

Z is O or S; and

R is methyl or ethyl;

which process comprises reacting a compound of formula (IV):

$X_1$-$COQ_1$ (IV)

with a compound of formula (V):

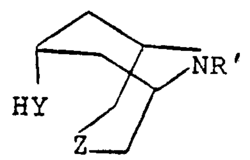

(V)

or a reactive derivative thereof, when Y is O; wherein $X_1'$ is $X_1$ or a group convertible thereto; $Q_1$ is a leaving group; $R'$ is R as defined in claim 1, or a hydrogenolysable protecting group; and the remaining variables are as defined in claim 3; and thereafter optionally converting $X_1'$ to $X_1$, including any $R_a$, $R_b$, $R_c$, $R_d$, $R_e$, $R_g$ or $R_{10}$, $R_{11}$, $R_{12}$, $R_{13}$, $R_{14}$ group to another such group, converting $R'$, when other than R, to R; and optionally forming a pharmaceutically acceptable salt of the resultant compound of formula (IA).

4. A process according to claim 3 wherein X is of sub-formula (a), one of $R_1$ and $R_3$ is hydrogen and $R_2$ and $R_4$ are both $C_{1-6}$ alkyl groups or are joined to form $C_{2-7}$ polymethylene.

5. A process according to claim 3 wherein X is of sub-formula (b), and $R_5$ is hydrogen or a methyl or ethyl group.

6. A process according to claim 3 wherein X is of sub-formula (d) and $R_7$ is methyl.

7. A process according to claim 3 wherein X is of sub-formula (f) wherein $R_{10}$ is methoxy, $R_{12}$ is amino and $R_{13}$ is chloro or bromo.

8. A process according to any one of claims 1 to 7, wherein Z is O.

9. A process according to claim 3, for the preparation of a compound selected from the group consisting of:

endo-4-amino-5-chloro-2-methoxy-N-(9-methyl-9-aza-3-oxabicyclo[3.3.1]nonan-7-yl)benzamide,
endo-N-(9-methyl-9-aza-3-oxabicyclo[3.3.1]nonan-7-yl)-N′-(2-methoxyphenyl)urea,
endo-N-(9-methyl-9-aza-3-oxabicyclo[3.3.1]nonan-7-yl)-1-methyl-1H-indazol-3-carboxamide,
endo-N-(9-methyl-9-aza-3-oxabicyclo[3.3.1]nonan-7-yl)-3,3-dimethyl-1-indoline carboxamide,
and pharmaceutically acceptable salts of any of the foregoing compounds.

10. Use of a compound of formula (I) or a pharmaceutically acceptable salt thereof, as defined in any one of claims 1 to 9, in the manufacture of a medicament for the treatment of migraine, cluster headache, trigeminal neuralgia, visceral pain, CNS disorders and/or emesis.